# EUROPEAN PATENT APPLICATION

(11) **EP 4 792 773 A1**
(43) Date of publication of application: **19.08.2026**
(21) Application number: 24877489.5
(22) Date of filing: 08.10.2024
(51) Int. Cl.: A61C 7/00, A61C 9/00, A61C 19/04, A61B 1/24, G06T 1/00, G06T 7/00, G16H 50/50, G16H 30/40, G16H 50/20, A61B 34/10

(54) **METHOD FOR DESIGNING ORTHODONTIC DEVICE THROUGH CORRECTION OF SET-UP TEETH MODEL**

(30) Priority: 12.10.2023 KR 20230136282
(71) Applicant: ODS Co. Ltd, Incheon 21990 (KR)
(72) Inventor: SIM, Miyoung, Incheon 21986 (KR); PARK, Sungwon, Incheon 21986 (KR)
(74) Representative: You Patent
(86) International application number: PCT/KR2024/015253
(87) International publication number: WO 2025/079952

(57) **Abstract**

The present invention relates to a method for designing an orthodontic appliance using a computer. The method comprises displaying a tooth arrangement image (S01), correcting an arrangement state of teeth in the tooth arrangement image (S02), correcting shapes of teeth in the tooth arrangement image (S03), displaying an orthodontic appliance on a screen according to the corrected tooth arrangement image (S04), and outputting the orthodontic appliance using a 3D printer (S05).

## Description

### TECHNICAL FIELD

The present invention relates to a method for designing an orthodontic appliance using a computer, and more particularly to a method for designing an orthodontic appliance capable of exerting a more efficient and accurate orthodontic force through correction of a tooth model.

More specifically, the present invention relates to a program that scans a patient's oral structure using a three-dimensional (3D) scanner to generate and store information relating to a treatment image (e.g., an image of the patient's tooth arrangement), performs a set-up by moving teeth on a screen, and designs an optimal orthodontic appliance according to the stored tooth image. According to the present invention, a more effective and precise orthodontic appliance design is output through a 3D printer.

Recently, methods using a 3D scanner to scan a patient's oral structure and store the information to produce a tooth model according to the stored information have been widely used. Such methods represent an improvement over conventional methods in which a dental impression is taken directly from a patient's teeth to produce a tooth model.

The present invention has a technical feature in that predetermined corrections are made to a set-up tooth image of a patient so that an orthodontic appliance displayed according to the tooth image can exert a more efficient and precise orthodontic force.

Ultimately, the present invention relates to a method for designing an orthodontic appliance using a computer, wherein an arrangement and shape of teeth are modified or corrected in advance before designing the orthodontic appliance so that an optimal orthodontic appliance can be designed according to various tooth arrangement states of patients requiring orthodontic treatment.

### BACKGROUND ART

A conventional method of manufacturing an orthodontic appliance is as follows.

First, a practitioner examines the patient's oral structure and produces a tooth model having the same shape as the patient's teeth. Generally, the patient's tooth model is produced by first creating an impression of the patient's oral structure and then forming the model using plaster or the like.

After such a plaster tooth model is produced, a sheet-type polyol material is thermally pressed from upper and lower directions using a forming machine to produce an orthodontic appliance customized for the patient. This conventional manufacturing method requires manual work through cooperation between a dentist and a dental technician and is performed by skilled dental technicians. As a result, a large amount of time and labor is required, which increases the manufacturing cost of orthodontic appliances.

The present invention is intended to produce an orthodontic appliance having a more efficient and precise orthodontic force by generating and storing tooth image information of a patient using a 3D scanner without taking a dental impression and then appropriately correcting the image using a computer.

Korean Patent No. 10-2489995 discloses a method for designing a transparent orthodontic appliance using a computer. The patented invention relates to a method for designing a transparent orthodontic appliance according to the patient's tooth arrangement while displaying the tooth arrangement image.

However, since the patented invention omits correction of the tooth arrangement, it may be difficult to secure an optimal orthodontic force for certain tooth arrangements, and thus it may be difficult to apply the invention to such tooth arrangements as they are.

Accordingly, the present invention is intended to overcome the above-described disadvantages by providing a method for designing an orthodontic appliance capable of obtaining a more efficient and precise orthodontic force by modifying or correcting the patient's tooth arrangement image itself before displaying the orthodontic appliance.

### Specific Instruction for Implementing the Invention

### Technical Problem

The method for designing an orthodontic appliance through correction of a tooth model according to the present invention has the technical objective of accurately identifying various oral conditions (tooth arrangement states) of patients, detecting problems in the dentition of each patient, and modifying the tooth model so that the orthodontic appliance can be designed to exert a more efficient and precise orthodontic force, thereby enabling optimal orthodontic treatment for the patient.

### Technical Solution

In order to achieve the above objective, the present invention provides a method for designing an orthodontic appliance using a computer, comprising:
displaying a tooth arrangement image (S01);
correcting an arrangement state of teeth in the tooth arrangement image (S02);
correcting a shape of teeth in the tooth arrangement image (S03);
displaying an orthodontic appliance on a screen according to the corrected tooth arrangement image (S04); and
outputting the orthodontic appliance using a three-dimensional (3D) printer (S05).

In the present invention, when a crown of a specific tooth is less than or equal to a preset value, the tooth is visually displayed according to a preset method and a margin line between the tooth and the gingiva is moved in a gingival direction. Alternatively, a specific margin line may be designated by a user, and the designated margin line is moved in the gingival direction to extend the crown of the tooth, thereby correcting the arrangement state of the tooth.

Further, the present invention includes identifying an active tooth to be orthodontically corrected and an inactive tooth for securing an orthodontic force for the active tooth, and adjusting a shape or volume of an undercut with respect to one or both of the active tooth and the inactive tooth to adjust the shape of the tooth.

Further, the present invention may include:
designating a crowding region (S033);
measuring a depth and width of a groove present in the designated crowding region (S034); and
setting the crowding region as a single tooth when the depth and width of the groove are less than or equal to a preset value (S035).

### EFFECTS OF THE INVENTION

The method for designing an orthodontic appliance through correction of a tooth model according to the present invention enables optimal orthodontic treatment by accurately identifying various oral conditions of patients.

In designing an orthodontic appliance to be output by a 3D printer, various corrections are performed on the tooth model in a prior stage so that the orthodontic appliance can have an optimal design.

The correction of the tooth model reflects various dentition states of individual patients, thereby enabling the design of an optimal orthodontic appliance and allowing the orthodontic force of the orthodontic appliance to be exerted most efficiently.

### BRIEF DESCRIPTION OF THE DRAWINGS

FIG. 1 illustrates a state in which an excessively large interproximal space is designated in a tooth arrangement to be corrected.
FIG. 2 illustrates a state in which an excessively small interproximal space is designated in a tooth arrangement to be corrected.
FIG. 3 illustrates a state in which an interproximal space is filled.
FIG. 4 illustrates a state in which a margin line of a tooth is moved toward the gingiva.
FIG. 5 illustrates a crowding state of teeth.

### Best Mode for Implementing the Invention

The present invention relates to a method for designing an orthodontic appliance using a computer, the method comprising:
displaying a tooth arrangement image (S01);
correcting an arrangement state of teeth in the tooth arrangement image (S02);
correcting a shape of teeth in the tooth arrangement image (S03);
displaying the orthodontic appliance on a screen according to the corrected tooth arrangement image (S04); and
outputting the orthodontic appliance using a three-dimensional (3D) printer (S05).

### Mode for Implementing the Invention

The present invention is not limited to the embodiments described below and may be implemented in various different forms. The embodiments described herein are provided only to make the disclosure of the present invention complete and to fully inform those skilled in the art to which the present invention pertains of the scope of the invention. The present invention is defined only by the scope of the claims.

The present invention relates to a method (or program) for designing an orthodontic appliance using a computer. Here, the term "computer" includes various devices capable of performing computational processing and providing results to a user, and generally includes an input unit, a processing unit, and an output unit.

In the present specification, an image relating to a tooth model refers to a multidimensional image, such as a two-dimensional or three-dimensional image, showing the overall arrangement of teeth, and includes all images obtained by medical imaging techniques using tomographic imaging. For example, such images may include CT (Computer Tomography) images, Nuclear Magnetic Resonance Computed Tomography (NMR-CT) images, Positron Emission Tomography (PET) images, Cone Beam CT (CBCT), and images obtained by an oral scanner.

In the present specification, a "tooth to be orthodontically corrected" refers to a tooth whose position is inappropriate or abnormally rotated and that requires movement of position and/or rotation through orthodontic treatment.

Hereinafter, embodiments of the present invention will be described in detail with reference to the accompanying drawings.

The present invention relates to a method for designing an orthodontic appliance using a computer.

The inside of a patient's oral cavity is scanned using a three-dimensional (3D) scanner, and the obtained dentition information of the patient is stored in a computer. A practitioner retrieves the patient's dentition information stored in the computer and displays it on an image display device such as a monitor.

Among the displayed teeth, the practitioner selects teeth to be orthodontically corrected and performs a process (set-up) of modifying the selected teeth into a corrected state. Through this process, a tooth arrangement image is completed, and an orthodontic appliance is designed according to the tooth arrangement image. Step S01 corresponds to displaying the tooth arrangement image.

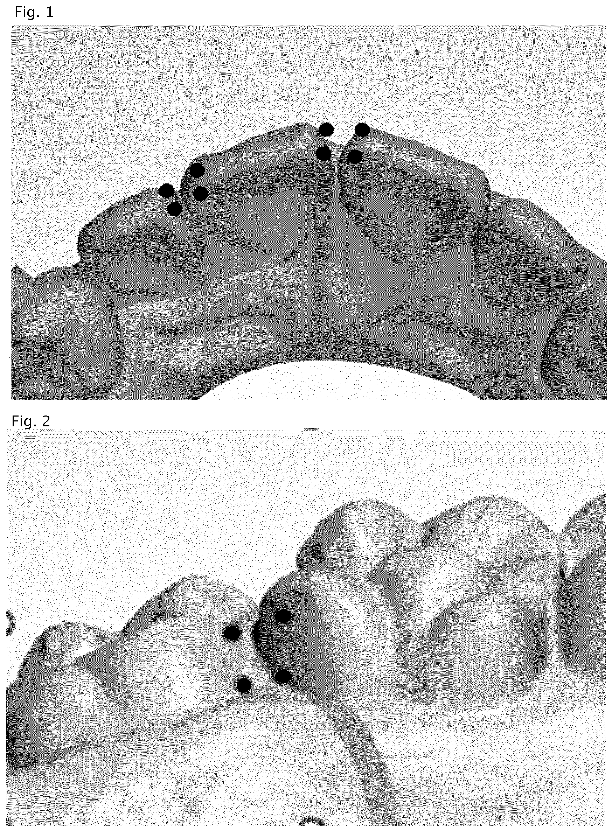

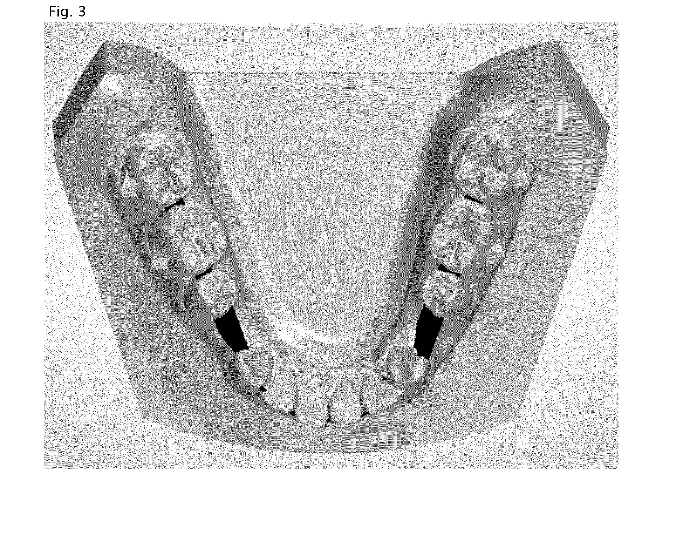

FIG. 1 illustrates an arrangement state of teeth to be corrected, in which an interproximal space larger than a predetermined value is designated. FIG. 3 illustrates a state in which the interproximal space has been corrected and filled.

Step S02 is a step of correcting the arrangement state of teeth in the tooth arrangement image. An orthodontic appliance is designed according to the arrangement state and outer surface shape of the teeth. Accordingly, an inner surface of the orthodontic appliance is generally formed to correspond to the outer surface of the teeth.

For this reason, if there is an unusual feature in the arrangement state of the teeth, structural problems may occur in the orthodontic appliance designed according to such arrangement (for example, the orthodontic appliance may become disconnected at an extracted region). The technical objective of the present invention is to prevent in advance the occurrence of structural problems in the orthodontic appliance caused by unusual characteristics in the arrangement state or shape of the teeth.

Therefore, when unusual characteristics exist in the arrangement state of teeth, the present invention includes a process of correcting the arrangement state of teeth in the tooth arrangement image (S02) or correcting the shape of teeth in the tooth arrangement image (S03). As shown in FIGS. 1 and 2, when an unusual characteristic exists in the tooth arrangement state, a user may mark the corresponding region by placing four points around the region. Alternatively, when an unusual characteristic exists in the tooth arrangement state, the corresponding region may be automatically visually displayed.

FIGS. 1 and 2 illustrate only an example in which the unusual region is marked with four points. However, this is merely one example of a display method, and various other visual methods may be used. Such variations should be regarded as simple design modifications and fall within the scope of the present invention.

The orthodontic appliance designed according to the present invention is completed by being output through a 3D printer. Before printing the orthodontic appliance, the design of the target tooth model must first be completed. After the design of the tooth model is completed and stored in the computer, a design operation for the orthodontic appliance is performed. The data relating to the completed orthodontic appliance is transmitted to a 3D printer and finally output. When the interproximal space of a tooth model exceeds an appropriate value, unnecessary portions may be additionally generated in the interior of the orthodontic appliance. Such additional portions may reduce the orthodontic force of the appliance and may cause inconvenience during attachment and removal of the orthodontic appliance, thereby reducing usability. For this reason, correction of the interproximal space is required.

According to the present invention, when a space between specific teeth is greater than or equal to a preset value, the interproximal space is visually displayed according to a preset method (such as four points, circular or rectangular borders, or color changes), and correction is performed by filling the interproximal space.

The filling of the interproximal space may be performed in various ways.

A practitioner may select a "fill" button from a menu. When the fill function is activated, the practitioner may operate a mouse to drag the corresponding region so that the region is appropriately filled. In this manner, a portion of the interproximal space may be partially designated and gradually filled on the screen. Various other methods may also be used for filling the interproximal space, and modifications that can be easily derived by those skilled in the art should be regarded as falling within the scope of the present invention.

The interproximal space designated by the practitioner may be visually displayed according to a preset method (e.g., four points, circular or rectangular borders, or color changes), and the interproximal space may be corrected by being automatically or manually filled. Although interproximal spaces having a value greater than or equal to a preset value may be automatically displayed, it is also possible for the practitioner to manually designate a specific interproximal space while observing the displayed tooth arrangement state. Such designated interproximal spaces may be corrected according to the method described above.

Although the correction of the tooth arrangement state described above has been explained with respect to interproximal spaces, the present invention is not limited thereto and may also be applied to cases where a space exists due to a missing tooth. When a specific tooth is missing, the corresponding space may be filled using the method described above.

As described above, the interproximal space may be filled gradually by a practitioner designating the region to be filled (e.g., an interproximal space). Alternatively, a filling material selected from a plurality of filling materials stored in advance in a computer may be positioned in the interproximal space to fill the space automatically.

For example, the practitioner may activate a "library" menu, in which various filling materials of different shapes and sizes are stored. The practitioner may select an appropriate filling material for the corresponding interproximal space. The shapes and sizes of the filling materials stored in the library may also be modified in real time, thereby increasing the ability to accommodate various interproximal spaces of different patients.

Meanwhile, although it may be advantageous for orthodontic treatment that one surface of the orthodontic appliance partially penetrates between teeth, correction of the tooth arrangement state may also be required when the interproximal space is too narrow for the orthodontic appliance to be formed as intended.

FIG. 2 illustrates another arrangement state of teeth to be corrected, in which an interproximal space smaller than a predetermined value is designated.

Particularly when orthodontic force must be secured in a distal or mesial direction of a tooth, it is effective for orthodontic treatment that a space exists between teeth so that the orthodontic appliance can be formed within the interproximal space.

When the interproximal space is too small and the space between teeth is excessively narrow, the interproximal space may be corrected by increasing its size, and the orthodontic appliance is displayed according to the corrected value.

The interproximal space may be enlarged by dragging a mouse or by inputting a specific numerical value. However, when the interproximal space is extremely narrow but securing orthodontic force is not required, correction of the interproximal space is not necessarily required.

Step S03 is a step of correcting the shape of teeth in the tooth arrangement image.

The step of correcting the shape of teeth (S03) includes corrections in cases where a portion of a tooth is damaged and a cavity is present, where the size of a tooth is too small, where a crown is too small, or where adjustment of an undercut size is required.

When a cavity is formed in a tooth, an unnecessary portion may be formed in the orthodontic appliance. Therefore, it is preferable that such cavities be removed. Cavities present in the tooth may be automatically displayed or may be visually identified by a practitioner. Once recognized, the cavity may be removed through a filling correction. Since the filling method is the same as that described above, a detailed description thereof will be omitted.

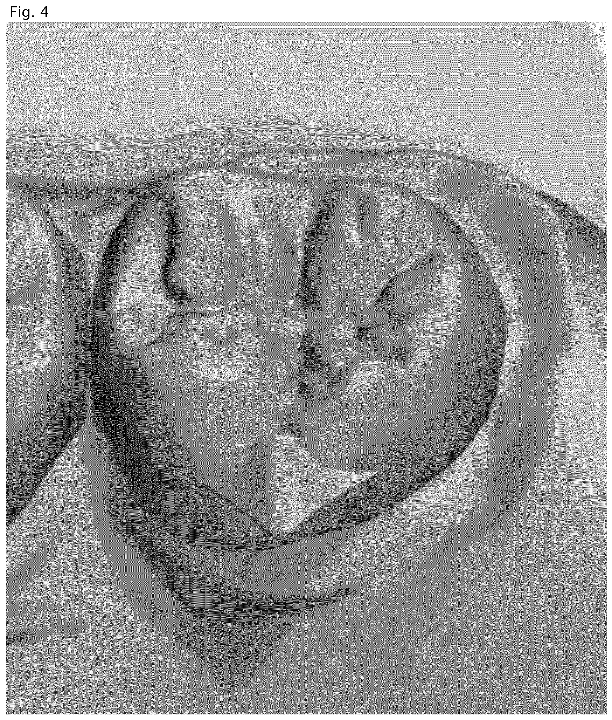

FIG. 4 illustrates a correction of a margin line of a tooth.

Meanwhile, when the size of a tooth is too small, a correction for increasing the size may also be required. For example, when the crown of a specific tooth is less than or equal to a preset value, the tooth may be automatically visually displayed according to a preset method. Alternatively, the practitioner may designate a specific crown that is determined to require correction while viewing the screen.

Thereafter, a correction may be performed in which a margin line between the tooth and the gingiva is moved in a gingival direction. The movement of the margin line in the gingival direction may be performed automatically or manually according to an operation by the practitioner.

When the margin line is moved in the gingival direction, the crown becomes larger, which results in an increase in the size (height) of the tooth. The amount by which the margin line is moved may be a preset value or may be a value designated by the practitioner in real time.

The correction of the margin line described above may be performed automatically, or a practitioner may designate a specific margin line and adjust the designated margin line in the gingival direction by a required length according to an operation by the practitioner.

By moving the margin line in the gingival direction to increase the size of the crown, the orthodontic appliance may be more stably supported on the corresponding tooth, thereby facilitating the securing of orthodontic force.

The present invention further includes identifying an active tooth to be orthodontically corrected and an inactive tooth for securing orthodontic force for the active tooth (S031), and adjusting the shape of an undercut with respect to one or both of the active tooth and the inactive tooth (S032). The undercut refers to a recessed portion of the tooth located at a boundary region between the tooth and the gingiva.

The active tooth refers to a tooth to be orthodontically corrected, and the inactive tooth refers to a tooth that functions as a support in order to secure orthodontic force. When the size of a tooth (e.g., area or height) is excessively small, it may be difficult to secure orthodontic force for the corresponding tooth. In such a case, the undercut of the tooth may be enlarged (i.e., the overall volume of the undercut may be increased) so that the orthodontic appliance can be more firmly attached to the tooth. A firm attachment of the orthodontic appliance indicates an increase in orthodontic force applied to the tooth.

The enlargement of the undercut may be performed for either the active tooth or the inactive tooth. In some cases (for example, when both teeth are small or when securing orthodontic force is particularly required), the enlargement may be applied to both.

Meanwhile, when the undercut of a tooth is excessively large (particularly when securing orthodontic force is not required), it is preferable to perform a correction by filling a portion or all of the unnecessary undercut. Even when sufficient orthodontic force can be secured, an excessively large undercut may cause difficulty in attaching or detaching the orthodontic appliance. Therefore, correction for reducing or removing the undercut is required because the orthodontic appliance is designed to fill the interior of the undercut.

The size of the undercut may be increased or decreased by filling a portion or all of the undercut or by increasing or decreasing the volume of the undercut itself.

The method of visually displaying an undercut when the volume of the undercut is greater than or less than a predetermined value, and the method of filling the undercut, are the same as those described above and therefore will not be described again. In addition, these processes may be automatically performed or may be manually performed by an operation of the practitioner, as described above.

After the correction process for the tooth arrangement image is completed, the method further includes displaying an orthodontic appliance on a screen according to the corrected tooth arrangement image (S04) and outputting the orthodontic appliance using a 3D printer (S05).

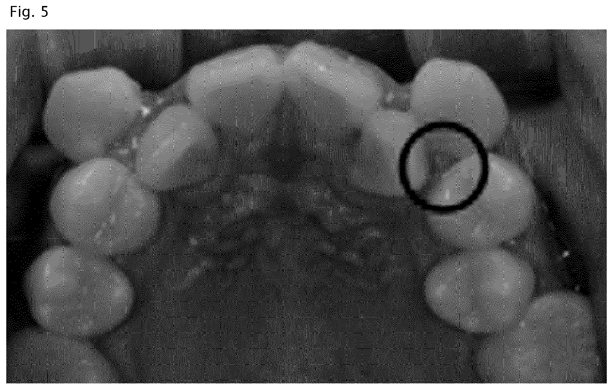

FIG. 5 is an image illustrating crowding of teeth.

The present invention further includes designating a crowding region (S033), measuring a depth and width of a groove present in the designated crowding region (S034), and setting the crowding region as a single tooth when the depth and width of the groove are less than or equal to a preset value (S035).

Teeth are generally arranged in a row; however, in some cases, multiple teeth form a cluster, which is referred to as crowding. Such crowding may cause significant obstacles in mounting the orthodontic appliance and securing orthodontic force.

The present invention proposes measuring the size (depth, width, etc.) of a groove located at a central portion of the crowding region and setting the entire crowding region as a single tooth when the measured values are less than or equal to a preset value. This is because recognizing each tooth included in the crowding region as an independent tooth may make the design of the orthodontic appliance extremely complex and difficult.

Of course, when the central space of the crowding region is relatively large, one surface of the orthodontic appliance (one of two surfaces that contact the outer and inner surfaces of the tooth) may be designed to pass through the central portion of the crowding region.

However, when the crowding is very dense and the groove is very small, it is preferable to assume that the crowding forms a single tooth and design the orthodontic appliance such that the outer surface and inner surface of the orthodontic appliance pass along the outer and inner sides of the crowding region (that is, along the overall outer boundary of the crowding region). In other words, in such a case, the orthodontic appliance is designed on the assumption that the crowding region constitutes a single tooth.

For each tooth included in the crowding region, shape correction, tooth alignment correction, undercut correction, and the like may also be performed. Since these corrections are the same as those described above, a detailed description thereof will be omitted.

The configuration and effects described above relate to one embodiment of the present invention and are not intended to limit the scope of the claims of the present invention. Various changes and modifications may be made without departing from the technical spirit of the present invention, and it will be apparent to those skilled in the art that such simple design modifications fall within the technical scope of the present invention.

## Claims

1. A method for designing an orthodontic appliance using a computer, comprising:
displaying a tooth arrangement image (S01);
correcting an arrangement state of teeth in the tooth arrangement image (S02);
correcting a shape of teeth in the tooth arrangement image (S03);
displaying an orthodontic appliance on a screen according to the corrected tooth arrangement image (S04); and
outputting the orthodontic appliance using a 3D printer (S05),
wherein the method is a method for designing an orthodontic appliance through correction of a tooth model.

2. The method of claim 1,
wherein in step S02,
when a specific inter-dental space is greater than or equal to a preset value, the inter-dental space is visually displayed according to a preset method, and correction is performed in a manner that the inter-dental space is filled.

3. The method of claim 1,
wherein in step S02,
when a specific inter-dental space is less than or equal to a preset value, the inter-dental space is visually displayed according to a preset method, and correction is performed in a manner that increases a size of the inter-dental space.

4. The method of claim 2,
wherein the filling of the inter-dental space is performed
by a method in which a portion of the inter-dental space is partially designated and gradually filled, or
by a method in which a filling material selected from a plurality of filling materials pre-provided in the computer is positioned in the inter-dental space so that the inter-dental space is filled.

5. The method of claim 1,
wherein in step S03,
when a crown of a specific tooth is less than or equal to a preset value,
the tooth is visually displayed according to a preset method, and a margin line between the tooth and gingiva is automatically moved in a gingival direction, or
a specific margin line is designated by an operator, and the designated margin line is moved in the gingival direction by an operation of the operator so that the crown of the tooth is extended.

6. The method of claim 1,
wherein step S03 comprises:
identifying an active tooth to be orthodontically corrected and an inactive tooth for securing orthodontic force for the active tooth (S031); and
adjusting a shape or volume of an undercut with respect to one or both of the active tooth and the inactive tooth (S032).

7. The method of claim 6,
wherein step S032 comprises
filling a portion or all of the undercut, or
decreasing or increasing the volume of the undercut.

8. The method of claim 1, further comprising:
designating a crowding region (S033);
measuring a depth and width of a groove present in the designated crowding region (S034); and
when the depth and width of the groove are less than or equal to a preset value, setting the crowding region as a single tooth (S035).
